# EUROPEAN PATENT APPLICATION

(11) **EP 1 538 218 A1**
(43) Date of publication of application: **08.06.2005**
(21) Application number: 03078853.3
(22) Date of filing: 04.12.2003
(51) Int. Cl.: C12Q 1/68

(54) **Method to diagnose or screen for inflammatory diseases**

(71) Applicant: Erasmus University Medical Center Rotterdam, 3015 GD Rotterdam (NL)
(72) Inventor: Drexhage, Hemmo, Arjan, 3051 JD Rotterdam (NL); Ruwhof, Cindy, 2401 PJ Alphen aan den Rijn (NL); Benner, Robert, 2992 SH Barendrecht (NL)
(74) Representative: Prins, Adrianus Willem, Mr. Ir.

(57) **Abstract**

The invention relates to the field of medical diagnostics. More specifically, the invention relates to methods to diagnose or screen for inflammatory condition or disease, including auto-inflammatory disease and affective disorder, in a subject, preferably a human subject, by assaying for a marker for an inflammatory disease. Provided is a method to diagnose or screen for an inflammatory disease in a subject, said method comprising determining the level of at least one, preferably at least two, more preferably at least three, most preferred at least four, inflammatory-specific gene product(s) in a biological sample isolated from said subject, preferably peripheral blood monocytes, wherein said inflammatory-specific gene is selected from the group comprising HSPC228, 34703_f_at, MCP-3, CCL2, EMP1, CDC42, TLE3, SPRY2, p40BBP, HSPC060, NAB2, HSPA1A, HSPA1B, MAPRE2,OAS1, CCR2, CX3CR1, DOK1, HBB, G-gamma globin, THBD, PHLDA1, DTR and GNLY.

## Description

The invention relates to the field of medical diagnostics. Among others, methods are provided to diagnose or screen for an inflammatory disease, amongst others for an auto-inflammatory disease, by assaying for an inflammatory disease-marker.

The immune system produces cytokines and other humoral factors to protect the host when threatened by inflammatory agents, microbial invasion, or injury. In most cases this complex defence network successfully restores normal homeostasis, but at other times the immunological mediators may actually prove deleterious to the host. Some examples of immune disease and immune system-mediated injury have been extensively investigated including anaphylactic shock, autoimmune disease, and immune complex disorders.

Recent advances in humoral and cellular immunology, molecular biology and pathology have influenced current thinking about inflammation and auto-immunity being a component of immune-mediated disease. These advances have increased our understanding of the basic aspects of antibody, B-cell, and T-cell diversity, the generation of innate (effected by monocytes, macrophages, granulocytes, natural killer cells, mast cells, γδ T cells, complement, acute phase proteins, and such) and adaptive (T and B cells and antibodies) or cellular and humoral immune responses and their interdependence, the mechanisms of (self)-tolerance induction and the means by which immunological reactivity develops against self- and non-self antigenic constituents. In general, T lymphocytes play a pivotal role in initiating the immune-mediated disease process (Sempe et al. 1991, Miyazaki et al. 1985, Harada et al. 1986, Makino et al. 1986). CD4+ T-cells can be separated into at least two major subsets T helper 1 and 2 (Th1 and Th2). Activated Th1 cells secrete IFN-γ and TNF-α, while Th2 cells produce IL-4, IL-5 and IL-10. Th1 cells are critically involved in the generation of effective cellular immunity, whereas Th2 cells are instrumental in the generation of humoral and mucosal immunity and allergy, including the activation of eosinophils and mast cells and the production of IgE (Abbas et al. 1996).

In general, immune-mediated disorders are difficult to treat. Often, broad-acting medication is applied, such as treatment with corticosteroids or any other broad-acting anti-inflammatory agent that in many aspects may be detrimental to a treated individual.

Since 1900, the central dogma of immunology has been that the immune system does not normally react to self. However, it recently become apparent that auto-immune responses are not as rare as once thought and that not all auto-immune responses are harmful; some responses play a distinct role in mediating the immune response in general. For example, certain forms of auto-immune response such as recognition of cell surface antigens encoded by the major histocompatibility complex (MHC) and of anti-idiotypic responses against self idiotypes are important, indeed essential, for the diversification and normal functioning of the intact immune system.

Apparently, an intricate system of checks and balances is maintained between various subsets of cells (i.e. T-cells) of the immune system, thereby providing the individual with an immune system capable of coping with foreign invaders. In that sense, auto-immunity plays a regulating role in the immune system.

However, it is now also recognised that an abnormal auto-immune response is sometimes a primary cause and at other times a secondary contributor to many human and animal diseases. Clinical and laboratory data of various types of auto-immune disease frequently overlap with each other and with other inflammatory disorders (e.g. transplant rejection, Alzheimer disease or due to trauma, burns or bacterial, viral or parasitic infection). More than one auto-immune and/or other inflammatory disorder tends to occur in the same individual, especially in those with auto-immune endocrinopathies. Auto-immune syndromes may be mediated with lymphoid hyperplasia, malignant lymphocytic or plasma cell proliferation and immunodeficiency disorders such as hypogammaglobulinaemia, selective Ig deficiencies and complement component deficiencies.

Examples of auto-immune diseases are: systemic lupus erythematosus (SLE), rheumatoid arthritis (RA), post-partum thyroid dysfunction, auto-immune thromocytopenia, psoriasis, scleroderma, dermatitis herpetiformis, polymyositis, dermatomyositis, pemphigus vulgaris, spondyloarthropathies such as vitiligo, ankylosing spondylitis, Sjögren's syndrome, multiple sclerosis (MS), Crohn's disease, myasthenia gravis, ulcerative colitis, autoimmune neuropathies, primary biliary cirrhosis such as Guillain-Barré, autoimmune hepatitis, autoimmune uveitis , Type 1 or immune-mediated diabetes mellitus (DM1), autoimmune hemolytic anemia, pernicious anemia, Grave's disease, autoimmune thrombocytopenia, Hashimoto's thyroiditis, autoimmune oophoritis and orchitis, autoimmune disease of the adrenal gland, Anti-phospholipid syndrome, vasculitides such as Wegener's granulomatosis, Behcet's disease. In addition to these more or less 'classical' examples of auto-immune disease, recent data indicate that some psychiatric or affective disorders also have an inflammatory component. Evidence supports that macrophages as well as lymphocytes and their products may be involved in the pathophysiology of affective disorders, major/unipolar depression, schizophrenia, seasonal affective disorder, post-partum depression, Alzheimer's disease, bipolar disorder (see Stasny et al. 2003; Pollmacher et al. 2002; Maes et al. 2001; Leu et al. 2001; Maes et al. 1995; Anisman et al. 2003; Leonard, 2001). Bipolar disorder, previously known as bipolar or manic depression, is a serious, double-edged mental illness. In contrast to the sustained bleakness of generalized depression (technically described as unipolar disorder), bipolar disorder is characterized by cyclical swings between elation and despair. It has been reported that the immune system is activated in bipolar patients. For example, the T cell system is activated in both symptomatic and euthymic patients with bipolar disorder (Breunis et al., " High numbers of circulating activated T cells and raised levels of serum IL-2 receptor in bipolar disorder." Biol Psychiatry. 2003;53(2):157). An earlier study showed that thyroid autoimmunity is highly prevalent in samples of outpatients with bipolar disorder (Kupka et al., Biol Psychiatry. 2002 15;51(4):305).

Auto-immune diseases are characterised by auto-immune responses, for example directed against widely distributed self-antigenic determinants, or directed against organ- or tissue specific antigens. Such disease may follow abnormal immune responses against only one antigenic target, or against many self antigens, or even due to trauma. In many instances, it is not clear whether auto-immune responses are directed against unmodified self-antigens or self-antigens that have been modified (or resemble) any of numerous agents such as viruses, bacterial antigens and haptenic groups.

There is as yet no established unifying concept to explain the origin and pathogenesis of the various auto-immune disorders. Studies in experimental animals support the notion that auto-immune diseases may result from a wide spectrum of genetic and immunological abnormalities which differ from one individual to another and may express themselves early or late in life depending on the presence or absence of many superimposed exogenous (viruses, bacteria, trauma) or endogenous (hormones, cytokines, abnormal genes) accelerating factors.

The diagnosis of (chronic) inflammatory and/or autoimmune disease is typically based on an individual's symptoms, findings from a physical examination, and results from laboratory tests. In some cases, a specific diagnosis can be made. A diagnosis shortly after onset of a patient's symptoms will allow for early aggressive medical therapy; and for some diseases, patients will respond completely to treatments if the reason for their symptoms is discovered early in the course of their disease. If an individual has skeletal symptoms such as joint pain and a positive but non-specific lab test, she or he may be diagnosed with the confusing name of early or "undifferentiated" connective tissue disease. In this case, a physician may want the patient to return frequently for follow up. The early phase of disease may be a very frustrating time for both the patient and physician. On the other hand, symptoms may be short-lived, and inconclusive laboratory tests may amount to nothing of a serious nature. Thus, autoimmune diseases are often difficult to diagnose, particularly early in the course of the disease. Symptoms of many autoimmune diseases―such as fatigue―are non-specific. Laboratory test results may help but are often inadequate to confirm a diagnosis.

In view of the above, there is a clear need for improved and alternative methods to diagnose, screen for, or predict (the development of) (chronic) inflammatory diseases, including auto-inflammatory diseases.

The invention now provides the insight that a number of genes are differentially expressed in patients suffering from an inflammatory disease when compared to healthy subjects. The differential expression level of 15 inflammatory-specific genes provides a basis for new clinically applicable tools to diagnose inflammatory disease, as well as to screen for patients who are at increased risk of developing an inflammatory disease.

Provided herein is a method to diagnose or screen for an inflammatory disease in a subject, preferably a human subject, said method comprising determining the level of at least one, preferably at least two, more preferably at least three, most preferred at least four, inflammatory-specific gene product(s) in a biological sample, preferably peripheral blood monocytes, isolated from said subject, wherein said inflammatory-specific gene product is selected from the group comprising HSPC228, 34703_f_a, MCP-3, CCL2, EMP1, CDC42, TLE3, SPRY2, p40BBP, HSPC060, NAB2, HSPA1A, HSPA1B, MAPRE2 and OAS1.

According to the invention, an elevated level of the HSPC228, 34703_f_at, MCP-3, CCL2, EMP1, CDC42, TLE3, SPRY2, p40BBP, HSPC060 and/or NAB2 gene product(s) and/or a reduced level of the HSPA1A, HSPA1B, MAPRE2 and/or OAS1 gene product(s) compared to the level of said gene product(s) in a sample of a healthy subject is indicative of an inflammatory disease or an increased risk of developing such a disease. Thus, a method is provided for the diagnosis or detection of an inflammatory disease in a subject by assaying for a marker of an inflammatory disease. Such a method is of very useful for diagnosing and/or monitoring the development of preclinical stages of such a disease in a patient and thus presents a valuable opportunity to initiate preventive or ameliorative treatment of the disease. The term "inflammatory disease" as used herein refers to various immune-mediated disorders, including chronic inflammatory disease, auto-immune disease, and affective disorders.

HSPC228 encodes a hypothetical protein, with the GenBank accession number M27826. 34703_f_at refers to zo30b03.r1 Stratagene colon (#937204) Homo sapiens cDNA clone IMAGE:588365 5' contains LTR7.b3 LTR7 repetitive element; mRNA sequence. The accession number of 34703_f_at is AA151971. MCP-3 refers to monocyte chemotactic protein 3, also known as chemokine (C-C motif) ligand 7 (CCL7) or small inducible cytokine A7 (SCA7). The accession number of MCP-3 is X72308. CCL2 refers to human JE gene encoding a monocyte secretory protein (exon 3), also known as chemokine (C-C motif) ligand 2, monocyte chemotactic protein 1 (MCP1), small inducible cytokine A2 (SCYA2) or monocyte chemotactic and activating factor (MCAF). The GenBank accession numbers of CCL2 are M28225 en M26683. EMP1 refers to epithelial membrane protein 1, with the GenBank accession numbers Y07909 and U43916. CDC42 refers to cell division cycle 42 (GTP-binding protein, 25kD). The accession number of CDC42 is M35543. TLE3 refers to transducin-like enhancer of split 3, homolog of Drosophila E (sp1). The GenBank accession number of TLE3 is M99438. SPRY2 refers to sprouty (Drosophila) homolog 2. The GenBank accession number of SPRY2 is AF039843. p40BBP refers to a protein which is likely to be an ortholog of rat brain specific binding protein. The accession number of p40BBP is AL080235. HSPC060 encodes a hypothetical protein and has the GenBank accession number AF150247. NAB2 refers to NGFI-A binding protein 2 (EGR1 binding protein 2). The accession number of NAB2 is X70991. HSPA1A and HSPA1B refer to the 70 kD heat shock protein (Hsp70) 1A and 1B, respectively. They are also known as MHC class III HSP70-1 and HSP70-2. The accession numbers are M11717 (HSPA1A) and M59830 (HSPA1B). MAPRE refers to microtubule-associated protein, RP/EB family, member 2. The accession number of MAPRE is X94232. OAS1 refers to Homo sapiens 2'-5' oligoadenylate synthetase gene, exon 8 and E18 isoform, complete cds, or to 2',5'-oligoadenylate synthetase 1 (40-46 kD). The accession numbers of OAS1 are M11810 and X04371.

In a preferred embodiment, determining the level of an inflammatory-specific gene product comprises determining the level of said product in an isolated sample from a subject suspected of (developing) an inflammatory disease relative to the amount of said specific gene product in a control sample. Suitable control samples include biological samples isolated from healthy subjects. It is also possible to determine the level of an inflammatory-specific gene product in a sample relative to the level of a housekeeping gene product in the same patient sample. Generally speaking, housekeeping genes are constitutively expressed genes which serve to maintain cellular function. As such, they are presumed to produce the minimally essential transcripts necessary for normal cellular physiology. With the advent of microarray technology, it has recently become possible to identify at least a "starter set" of housekeeping genes, as exemplified by the work of Velculescu *et al*. (Nat. Genet 23: 387-388, 1999) who examined the expression of 7,000 full-length genes in 11 different human tissues, both adult and fetal, to determine the suite of transcripts that were commonly expressed throughout human development and in different tissues. They identified 535 transcripts via microarray hybridization as likely candidates for housekeeping genes, also referred to as "maintenance" genes. Preferably however, a control sample comprises (a defined amount of) an isolated inflammatory-specific gene product, such as a vector with an inflammatory-specific gene, purified RNA of an inflammatory-specific gene, or protein encoded by an inflammatory-specific gene.

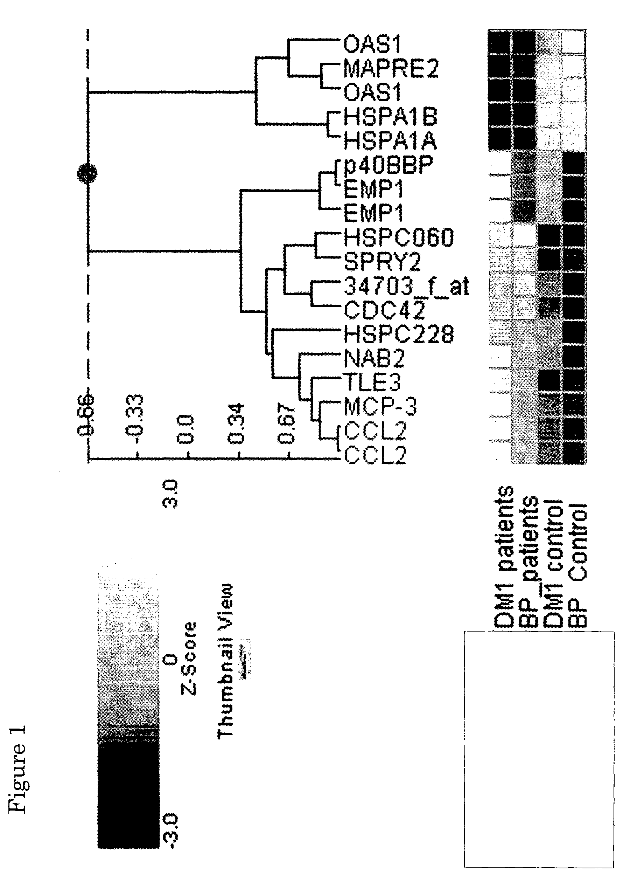

In one embodiment of the invention, the level of an inflammatory-specific gene product is determined at the DNA or RNA level, preferably at the mRNA level. As is exemplified in the Detailed Description, the expression profile of human monocytes isolated from subjects suffering from different inflammatory diseases (diabetes mellitus Type 1 and Bipolar depression) was compared to the expression profile in monocytes of healthy subjects using a U95Av2 GeneChip microarray from Affymetrix. The U95Av2 GeneChip contains over 12,600 transcripts of known genes and expressed sequence tags (ESTs). Surprisingly, it was found that the expression level of 15 human genes was specifically altered in patients with an inflammatory disease. The term '"inflammatory-specific gene product" as used herein refers to a product (nucleic acid sequences as well as proteinaceous substances) which is encoded by one of these 15 genes. The expression level of four genes (HSPA1A, HSPA1B, MAPRE2 and OAS1) was reduced in patients with an inflammatory disease, whereas the expression of eleven genes (HSPC228, 34703_f_at, MCP-3, CCL2, EMP1, CDC42, TLE3, SPRY2, p40BBP, HSPC060, NAB2) was elevated in these patients. For example, the so-called heatmap in Fig. 1 shows that CCL2 is highly expressed in patients who suffer from either Type 1 diabetes mellitus (DM1) or bipolar depression (BP), when compared to age-matched DM1 or BP controls. Also, whereas HSPC228 is only expressed at a relatively low level in patients, HSPC228 expression is much lower in controls. In contrast, the expression of for example HSPA1A and HSPA1B are clearly reduced in both DM1 and BP patients when compared to controls. Thus, evaluation of the expression level of at least one of these 15 inflammatory-specific genes ("inflammatory-markers") allows to diagnose or screen for an inflammatory disease, because an up-regulation of HSPC228, 34703_f_at, MCP-3, CCL2, EMP1, CDC42, TLE3, SPRY2, p40BBP, HSPC060 or NAB2, or a down-regulation of HSPA1A, HSPA1B, MAPRE2 and OAS1 expression is only found in patients and not in healthy subjects (Figure 1). Herewith, we have provided diagnostic markers for inflammatory disease, in particular auto-inflammatory or autoimmune disease. In a further aspect of the invention, these markers are advantageously used as prognostic markers because they also have prognostic value with regard to disease development and/or complications, e.g. after trauma or infectious disease (e.g. Guillain-Barré syndrome). To this end, appropriate cell samples of such a subject can be cultured *in vitro* with an appropriate stimulus, e.g. a lectin, lipopolysaccharides or stimulating antibody. Using the markers provided herein, it is possible to discriminate between different conditions on the basis of different expression patterns. A specific combination of differentially expressed genes can be indicative of (an increased risk of developing) a specific disease or condition, for example in response to trauma or a bacterial, viral or parasitic infection. Furthermore, determination of one or more genes with a dysregulated expression level can be used to determine the stage or severity of an inflammatory disorder or disease.

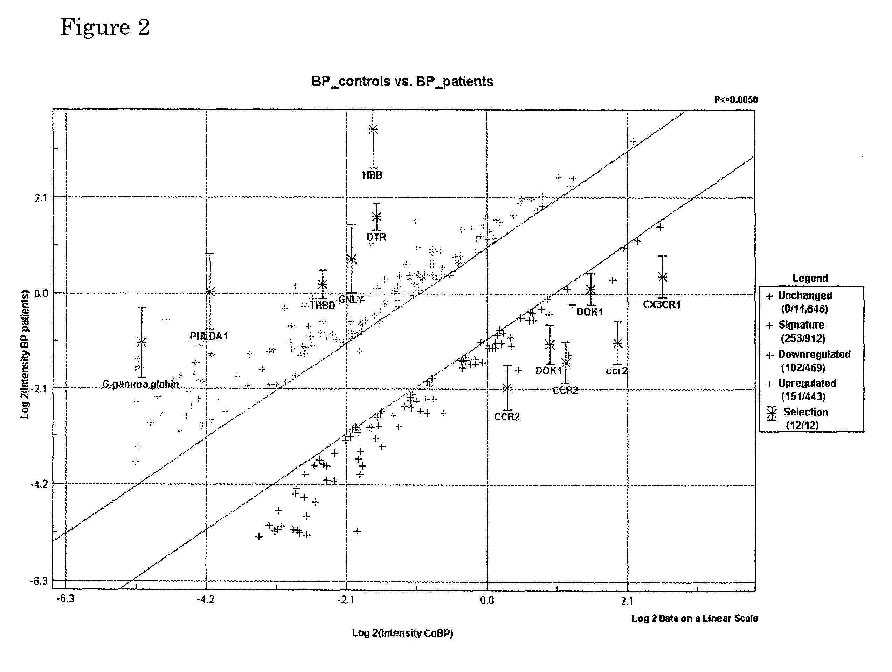

Next, using the same GeneChip approach as described above, genes were identified that were specifically up- or down-regulated in patients with a major affective disorder (in this case bipolar disorder (BP)), but not in DM1 patients or age-matched controls. As is depicted in Fig. 2, the expression level of CCR2, CX3CR1 and DOK1 is reduced in BP patients when compared to age-matched controls, whereas an increased expression was observed for HBB, G-gamma globulin, THBD, PHLDA1, DTR and GNLY. CCR2 refers to the chemokine (C-C motif) receptor 2. The GenBank accession numbers of CCR2 are U03905, U95626 and U03905. CX3CR1 refers to the chemokine (C-X3-C) receptor 1, with the accession number U20350. DOK1 refers to the 62 kD docking protein 1 (reported to act downstream of tyrosine kinase 1). The accession numbers of DOK1 are AF035299 and U70987. HBB refers to hemoglobin beta. HBB expression in BP patients was approximately 60-fold increased when compared to healthy controls. The accession number of HBB is M25079. G-gamma globulin refers to *Homo sapiens* G-gamma globulin (G-gamma globin) and A-gamma globin genes, complete cds. The accession number of G-gamma globulin is M91036. THBD refers to human thrombomodulin gene, complete cds. The accession number of THBD is J02973. PHLDA1 refers to pleckstrin homology-like domain, family A, member 1. The accession number of THBD is J02973. DTR refers to diphtheria toxin receptor (heparin-binding epidermal growth factor-like growth factor). The accession number of DTR is M60278. GNLY refers to granulysin, with the accession number M85276. Herewith, the invention provides a method to diagnose or screen for affective disorder (AD) in a subject, preferably a human subject, said method comprising determining the level of at least one, preferably at least two, more preferably at least three, most preferred at least four, -AD-specific gene product(s) in a biological sample isolated from said subject, preferably peripheral blood monocytes, wherein said gene is selected from the group comprising CCR2, CX3CR1, DOK1, HBB, G-gamma globin, THBD, PHLDA1, DTR and GNLY. In one embodiment, said detection is performed at the mRNA level. Preferably, the increase in the HBB, G-gamma globin, THBD, PHLDA1, DTR and/or GNLYmRNA level is at least two-fold compared to the level of said mRNA in a sample of a healthy subject to be indicative of AD. Likewise, the decrease in the CCR2, CX3CR1 and/or DOK1 mRNA level is at least two-fold compared to the level of said mRNA in a sample isolated from a healthy subject. More preferred, the altered expression level of either one of these AD-specific genes according to the invention is more than three-fold, or more than four-fold, or even higher than that, when compared to controls.

Detection of an inflammatory-specific or affective disorder-specific nucleic acid sequence can be achieved by conventional techniques well known in the art. These include PCR techniques for detecting specific DNA sequences, and reverse transcriptase (RT) PCR techniques for detecting specific RNA sequences. In one embodiment, a specific gene product is detected using a nucleic acid amplification assay, preferably a PCR assay, using a set of nucleic amplification primers capable of specifically amplifying said gene product. More preferred, said PCR assay is a real-time quantitative PCR (RQ-PCR) assay. The simplest RQ-PCR technique is based on detection of PCR products by the DNA-intercalating dye SYBR Green I. Other suitable RQ-PCR analyses involve those using fluorochrome-labeled sequence-specific probes including hydrolysis probes or hybridisation probes.

In a preferred embodiment of the invention, the increase in the HSPC228, 34703_f_at, MCP-3, CCL2, EMP1, CDC42, TLE3, SPRY2, p40BBP, HSPC060, NAB2, CCL2, MCP-3, EMP1, STX1A, CD9, PTPN7, CDC42, FABP5 and/or NAB2 mRNA level is at least two-fold compared to the level of said mRNA in a sample of a healthy subject to be indicative of an inflammatory condition or disease. Likewise, the decrease in the HSPA1A, HSPA1B, MAPRE2 and/or OAS1 mRNA level is at least two-fold compared to the level of said mRNA in a sample of a healthy subject. More preferred, the altered expression of either one of these inflammatory-specific genes is more than three-fold, or even more than four-fold, or even higher than that, when compared to control.

A method of the invention is easily practiced in routine laboratory settings. Specific gene products according to the invention can be analysed in various types of cells which are easily obtained from a subject, including monocytes, dendritic cells, T cells, granulocytes, natural killer T cells and other (natural) killer cells. All that is required is obtaining or isolating a sample from the subject, preferably a peripheral blood sample, isolating the cell type of interest (preferably monocytes), optionally preparing an extract of the sample (e.g. nucleic acid extract or a total cell lysate) and determining the level of a specific gene product in the sample or in an extract thereof. The level can be determined by contacting a cellular component of the sample with at least one reagent specifically reactive with an inflammatory-specific gene product and, optionally, with at least one reagent specifically reactive with a housekeeping gene product. The reagent or reagent(s) may be labeled and they may be immobilized on a solid support, for example a glass, nylon or nitrocellulose solid support. Also provided are reagents, such as nucleotide probes and antibodies specifically reactive with an inflammatory-specific gene product or with a fragment thereof.

In another embodiment, a nucleic acid extract of a sample isolated from a subject is contacted with at least one nucleotide probe comprising a sequence that hybridises to a nucleotide region encoding an inflammatory- or BP-specific gene and, optionally, with at least one nucleotide probe comprising a sequence that hybridises to a nucleotide region encoding a housekeeping gene. Said at least one nucleotide probe may be immobilized on a solid support, for example in an array format. Examples of suitable nucleotide probes comprise DNA, RNA or cDNA probes. The nucleic acid extract may contain nucleic acids with a detectable label, e.g. biotinylated cRNA.

Different types of DNA microarrays for use in a method of the invention can be prepared according to methods known in the art, e.g. arrays based on standard microscopic glass-slides on which cDNAs or long oligonucleotides (typically 70-80-mers) have been spotted. An other type is based on photolithographic techniques to synthesize 25-mer oligonucleotides on a silicon wafer and constitutes the patented Affymetrix technology.

In a further embodiment, a method to diagnose or screen for an (auto) inflammatory condition or disease or affective disorder in a subject comprises determining the level of at least one inflammatory- or - specific gene product at the protein level. To this end, an extract is prepared from a sample isolated from a subject which allows the specific detection of a protein, for instance by preparing a total cell lysate in the presence of a chaotropic agent such as a detergent or a salt. A sample (comprising at least one cell) from the subject may be pretreated prior to preparing an extract to improve detection of an inflammatory-specific gene product. For example, some proteins including CCL2 and MCP-3 are known to be rapidly secreted following their synthesis. Pretreatment with a protein secretion inhibitor (e.g. monensin) can thus be used to accumulate a specific protein within the cell cytoplasm and therewith increase the content of one (or more) inflammatory-specific gene products in an extract. An extract of a sample can subsequently be contacted with a specific binding partner of at least one protein (or fragment thereof) encoded by an specific gene product under conditions that allow formation of a complex between said binding partner and said protein and detecting the complex formation. The amount of complex formed is indicative of the amount of said inflammatory-specific protein present in the sample extract. Therefore, it is advantageous to use a binding partner that is provided with a detectable label, such as a fluorochrome, radioactive label, enzyme etc. A well-known method known in the art to specifically detect a protein involves the immunological detection of a protein using a specific (monoclonal or polyclonal) antibody or fragment thereof (e.g. single chain Fv) as a specific binding partner. Monoclonal antibodies may be obtained by well-established methods, e.g. as described in A. Johnstone and R. Thorpe, Immunochemistry in Practice, 2nd. Ed., Blackwell Scientific Publications, 1987, pp. 35-43. When prepared by recombinant DNA techniques, the antibody may be produced by cloning a DNA sequence coding for the antibody or a fragment thereof into a suitable cell, e.g. a microbial, plant, animal or human cell, and culturing the cell under conditions conducive to the production of the antibody or fragment in question and recovering the antibody or fragment thereof from the culture. Possible strategies for the preparation of cloned antibodies are discussed in, for instance, L. Riechmann et al., Nature 332, Mar. 24, 1988, p. 323 ff., describing the preparation of chimeric antibodies of rat variable regions and human constant regions; M. Better et al., Science 240, May 20, 1988, p. 1041 ff., describing the preparation of chimeric mouse-human Fab fragments; A. Sharra and A. Pluckthun, Science 240, May 20, 1988, pp. 1038-1040, describing the cloning of an immunoglobulin Fv fragment containing antigen-binding variable domains; and E. S. Ward et al., Nature 341, Oct. 12, 1989, pp. 544-546, describing the cloning of isolated antigen-binding variable domains ("single-domain antibodies").

In one aspect of the invention, a method is provided to diagnose or screen for, or predict the development of an inflammatory condition or disease in a subject wherein said method comprises the immunological detection of at least one, preferably at least two, more preferably at least three inflammatory-specific protein(s) in a biological sample isolated from said subject, wherein said protein is encoded by a gene selected from the group comprising HSPC228, 34703_f_at, MCP-3, CCL2, EMP1, CDC42, TLE3, SPRY2, p40BBP, HSPC060, NAB2, HSPA1A, HSPA1B, MAPRE2 and OAS1. In a second aspect, a method is provided to diagnose or screen for, or to predict the development of an affective disorder in a subject wherein said method comprises the immunological detection of at least one, preferably at least two, more preferably at least three AD-specific protein(s) in a biological sample isolated from said subject, wherein said protein is encoded by a gene selected from the group comprising CCR2, CX3CR1, DOK1, HBB, G-gamma globin, THBD, PHLDA1, DTR and ONLY.

Immunological detection is a common tool used in discovering protein expression patterns, isolating proteins from cellular extracts and in screening cells and extracts for specific proteins. The immunological detection in a method of the invention can be performed according to well known assays, including Western blotting, immunoprecipitation assays, enzyme-linked immunosorbant assays (ELISA), dot-blot assays and chip-based assays. Detailed practical information regarding the immunological detection of proteins can be found in "Antibodies: A Laboratory Manual" by Ed Harlow and David Lane (Editors) ISBN: 0879695447, Publisher: Cold Spring Harbor. In a specific aspect of the invention, an inflammatory-ELISA test is provided which is based on the principle of a solid phase enzyme-linked immunosorbant assay. As an example, a CCL2-ELISA test is described. The assay system utilizes a monoclonal antibody directed against an antigenic determinant of CCL2 that is used for solid phase immobilization (e.g. in microtiter wells). A goat antibody conjugated to horseradish peroxidase (HRP) reactive with a different antigenic determinant of CCL2 is present in an antibody-enzyme conjugate solution. The test sample (e.g. cell lysate) is allowed to react simultaneously with the two antibodies, resulting in the CCL2 molecule being sandwiched between the solid phase and enzyme-linked antibodies. After a 1 hour incubation at room temperature, the wells are washed with water to remove unbound labeled antibodies. A solution of HRP-substrate, for instance 3,3',5,5'-tetramethylbenzidine (TMB), is added and incubated for 20 minutes, resulting in the development of a blue colour. The colour development is stopped with the addition of a stop solution (e.g. 1 N HCl) changing the colour to yellow. Absorbance is measured spectrophotometrically at 450 nm. The concentration of CCL2 is directly proportional to the colour intensity of the test sample. An increase in the concentration of CCL2 in a sample is indicative of an inflammatory condition or disease. Of course it is preferred to include a standard curve of known amounts of the CCL2 protein in order to quantitate the amount of CCL2 present. Furthermore, the detection of a protein which is not subject to a change in patients with an (auto) inflammatory disease, for example a housekeeping enzyme, may be used as internal control for the amount of protein assayed and/or as a control between different samples. In a similar fashion, an AD -ELISA test can be designed.

In a further embodiment, a test kit is provided for diagnosing or screening for an inflammatory disease, such as Diabetes Mellitus Type 1 (DM1) or affective disorder, in a subject comprising at least one reagent specifically reactive with a gene product selected from the group comprising HSPC228, 34703_f_at, MCP-3, CCL2, EMP1, CDC42, TLE3, SPRY2, p40BBP, HSPC060, NAB2, HSPA1A, HSPA1B, MAPRE2 and OAS1. Said kit optionally further comprises at least one reagent specifically reactive with a housekeeping gene product. A suitable reagent can be an antibody or fragment thereof, or a nucleotide probe, specifically reactive with an inflammatory-specific gene product. Said reagent may be immobilized on a solid support, preferably a glass, nylon or nitrocellulose solid support. The physical shape of the solid support is not critical, although some shapes may be more convenient than others for the present purpose. Thus, the solid support may be in the shape of a plate, e.g. a microtiter plate, or a paper strip, dipstick, membrane (e.g. a nylon membrane or a cellulose filter) or solid particles (e.g. latex beads). Furthermore, a kit preferably also comprises a defined amount of one or more inflammatory-specific gene products which can serve as a control sample. A vector with such a specific gene, (purified) RNA of a specific gene, or (purified) protein encoded by an inflammatory-specific gene can be used as a quantitative control.

For example, a test kit is provided comprising an array of nucleotide probes comprising at least 10 nucleotide bases in length, wherein at least one probe hybridises to a fragment of at least one inflammatory-specific gene selected from the group comprising HSPC228, 34703_f_at, MCP-3, CCL2, EMP1, CDC42, TLE3, SPRY2, p40BBP, HSPC060, NAB2, HSPA1A, HSPA1B, MAPRE2 and OAS1, and optionally, wherein at least one probe hybridises to a fragment of at least one housekeeping gene. Of course, the different probes should be specific for the different inflammatory-specific genes. Said array of nucleotide probes is for instance arranged on a solid support in multiple discrete regions of distinct nucleic acid strands. In another example, a kit according to the invention to diagnose or screen for an inflammatory disorder or disease comprises one or more antibodie(s) specifically reactive with an inflammatory-specific protein. Said antibodies can be labelled to aid in detection of an antibody-antigen complex. The label substance for the reagents is preferably selected from the group consisting of enzymes, coloured or fluorescent substances and radioactive isotopes.

Examples of enzymes useful as label substances are peroxidases (such as horseradish peroxidase), phosphatases (such as acid or alkaline phosphatase), β-galactosidase, urease, glucose oxidase, carbonic anhydrase, acetylcholinesterase, glucoamylase, lysozyme, malate dehydrogenase, glucose-6-phosphate dehydrogenase, β-glucosidase, proteases, pyruvate decarboxylase, esterases, luciferase, etc. Enzymes are not in themselves detectable but must be combined with a substrate to catalyse a reaction the end product of which is detectable. Thus, a substrate may be added after contacting the support with the labelled reagent, resulting in the formation of a coloured or fluorescent substance. Examples of substrates which may be employed according to the invention include hydrogen peroxide/tetramethylbenzidine or chloronaphthole or o-phenylenediamine or 3-(p-hydroxyphenyl) propionic acid or luminol, indoxyl phosphate, p-nitrophenylphosphate, nitrophenyl galactose, 4-methyl umbelliferyl-D-galactopyranoside, or luciferin. Alternatively, the label substance may comprise coloured or fluorescent substances, including Europium, gold particles, coloured or fluorescent latex particles, dye particles, fluorescein, phycoerythrin or phycocyanin. Radioactive isotopes which may be used for the present purpose may be selected from I-125, I-131, H-3, P-32, P-33 and C-14.

Preferably, a kit further comprises standard amounts of (lyophilised) inflammatory-specific protein and, optionally, other reagents required for immunological detection of a protein such as a binding buffer, enzyme substrate, stop solution, and the like. An inflammatory-specific protein may be obtained using recombinant expression of a nucleic acid sequence encoding said inflammatory-specific protein in a host cell, preferably followed by purification of the protein. Of course, in a similar fashion, using AD-specific genes, test kits and arrays can be designed to diagnose, screen for or to predict affective disorder.

A method of the invention, a kit as defined above or an array of probes is advantageously used in the diagnosis or screening for increased risk of developing an (auto)inflammatory disease or an affective disorder in a subject, preferably a human subject.

### Example

### Experimental Procedures

### Subjects

Peripheral blood mononuclear cells (PBMCs) were isolated from heparinized peripheral blood using Ficoll-Paque (Amersham Biosciences, Uppsala, Sweden) density gradient centrifugation, resuspended in RPMI1640 medium containing HEPES and ultraglutamin supplemented with 10% fetal calf serum and 10% DMSO, and stored at -180 °C providing a bank for experiments. This study was approved by the Medical Ethical Committee of the Erasmus MC, Rotterdam, The Netherlands. All subjects provided written informed consent prior to participation.

At the start of an experiment, the PBMCs were quickly thawed and pooled into patient samples or control samples to minimize individual variations. 7 Bipolar Disorder (BP) patients were pooled into BP samples ( 2 different pools; mean age 42.1 years), 7 healthy controls for BP patients (CoBP; sex- and age-matched) into CoBP samples (2 different pools). 8-9 recent onset type 1 Diabetes Mellitus (DM1) patients were pooled into DM1 samples (3 different pools; mean age 14.4 years), 7-8 healthy controls for DM1 patients (CoDM1; age-matched) into CoDM1 samples (3 different pools).

The pooled cell samples were labeled with anti-CD 14 magnetic beads (Miltenyi Biotec GmbH, Bergisch Gladbach, Germany) for 20 minutes at 4 °C and separated into CD14+ and CD 14- fractions using the autoMACS (Miltenyi Biotec), program positive selection sensitive. The CD 14+ fraction represents the monocytes with a purity of at least 94%.

### RNA isolation

Total RNA was extracted from the monocytes with the use of a RNeasy Mini kit and QIAshredders from Qiagen (Westburg, Leusden, The Netherlands) according to the manufacturer's instructions. The integrity of the RNA was tested on 1.2% formaldehyde containing agarose gels.

### Target preparation

DNA complementary to the total RNA samples was synthesized from 4-5 µg of total RNA using an Superscript double-Stranded Synthesis kit from Invitrogen (Breda, The Netherlands) and a T7-(dT)24 primer (GenSet Oligos, Paris, France) according to the manufacturer's instructions. The cDNA served as a template for in vitro transcription reaction (37 °C for 5 h) using T7 RNA polymerase and biotinylated ribonucleotides employing an Enzo BioArray High Yield RNA transcript labeling kit (Enzo Life Sciences, Farmingdale, New York, USA). The cDNA and cRNA was purified using the GeneChip Sample Cleanup module (Affymetrix, Santa Clara, California, USA). The cRNA was quantified by spectrophotometric methods. An adjusted cRNA yield was calculated to reflect carryover of unlabeled total RNA.

### Hybridization and Staining

Twenty micrograms of biotinylated cRNA was randomly fragmented by heat and alkaline treatment. To check the quality of the procedure 5 µg cRNA was hybridized to a Test3 microarray (Affymetrix). Subsequently 10 µg of cRNA was hybridized to an U95Av2 microarray (Affymetrix; HG-U95Av2 GeneChip) for 16 h at 45 °C. The U95Av2 GeneChip contains 12,600 transcripts of known genes and expressed sequence tags (ESTs) that are characterized on their function- or disease-association. After washing and staining with PE-conjugated streptavidin, the microarrays were scanned in an HP/Affymetrix scanner at 570 nm using a krypton/argon laser.

### Data analysis

Image analysis was performed with Microarray Suite, version 5.0 (Affymetrix) using the control sample as baseline. To facilitate comparison between samples and experiments, the trimmed mean signal of each microarray was scaled to a target intensity of 2500. The expression profiles of DM1 and BP were compared to expression profiles of the CoDM1 and CoBP, respectively. The expression profiles were exported to the Rosetta Resolver Expression Data Analysis System (Rosetta Inpharmatics, Kirkland, Washington, USA) for further analyses including comparison analyses and cluster analyses. To increase confidence, the three DM1 profiles, three CoDM1 profiles, two BP profiles, and two CoBP profiles were combined in an error weighted fashion into a single DM1 experiment, a single CoDM1 experiment, a single BP experiment, and a single CoBP experiment, respectively. The data of these experiments were used to create the intensity-based ratio experiments DM1 versus CoDM1 and BP versus CoBP. The genes that were changed at least 4-fold between all patients and their controls and had a p≤0.01 in these ratio experiments were grouped together as biosets (a DM1 bioset and a BP bioset). We then clustered data of the DM1, CoDM1, BP, and CoBP, experiments restricted to the genes present in both the biosets employing the agglomerative clustering algorithm. The results of this 2D cluster was displayed in a heatmap in which each cell represents a log(ratio). This heatmap was used to select inflammatory-specific genes, that is genes that were up-or downregulated in DM1 and BP patients, but not in CoDM1 nor in CoBP patients (see Figure 1).

To identify BP specific genes, the genes present in the BP bioset but not present in the DM1 bioset were further analyzed. The genes that 1) were changed at least 4-fold between BP patients and their controls; 2) had a clear detectable signal (Rosetta recalculated intensity of at least 1.0) in the BP patients or in the CoBP; and 3) had a p≤0.01 in the BP versus CoBP ratio experiment, were grouped together as a new bioset (BP-specific bioset). We then plotted the intensity of the selected genes (of BP-specific bioset) in the BP patients against the intensity of those genes in BP controls (see Figure 2).

### Legends

Figure 1: Microarray analysis of DM1 patients, PB patients, DM2 patients and their sex- and age-matched controls. Purified monocytes were pooled and analyzed for gene expression using DNA microarrays. The relative expression levels of the selected inflammatory-specific genes across DM1 patients, DM1 controls, BP patients and BP controls are shown in a heatmap. Intensities were normalized across all intensity experiments and expressed on a gray scale by use of the software Rosetta Resolver. The increase (white) and decrease (black) in expression level relative to the median value are shown for the 6 experiments. The genes were also clustered using agglomerative clustering algorithm. Rows represent the relative intensity in the six groups. Columns represent the genes. Each cell in the heatmap represents a Z-score.
Figure 2: Scatter plot of intensity of genes in BP patients (Y-axis) versus sex-and age-matched BP controls (X-axis) is shown. Expression levels were averaged (weighted on basis of error) across two replicate experiments.

### REFERENCES

Abbas AK, Murphy KM, Sher A. Nature. 1996 Oct 31;383(6603):787-93. "Functional diversity of helper T lymphocytes."
Anisman H, Merali Z. Ann Med. 2003;35(1):2-11. "Plasma-soluble interleukin-2 and transferrin receptor in schizophrenia and major depression.
Cytokines, stress and depressive illness: brain-immune interactions."
Leonard BE. Int J Dev Neurosci. 2001 Jun;19(3):305-12. "Changes in the immune system in depression and dementia: causal or co-incidental effects?"
Leu SJ, Shiah IS, Yatham LN, Cheu YM, Lam RW. J Affect Disord. 2001 Mar;63(1-3):27-34. "Effects of pregnancy and delivery on the availability of plasma tryptophan to the brain: relationships to delivery-induced immune activation and early post-partum anxiety and depression."
Maes M, Meltzer HY, Buckley P, Bosmans E. Eur Arch Psychiatry Clin Neurosci. 1995;244(6):325-9. "Immune-inflammatory markers in patients with seasonal affective disorder: effects of light therapy."
Maes M, Ombelet W, Verkerk R, Bosmans E, Scharpe S. Psychol Med. 2001 Jul;31(5):847-58. Brain Behav Immun. 2002 Oct; 16(5):525-32. "Low levels of circulating inflammatory cytokines--do they affect human brain functions?"
Pollmacher T, Haack M, Schuld A, Reichenberg A, Yirmiya R. Brain Behav Immun. 2002 Oct;16(5):525-32. "Low levels of circulating inflammatory cytokines--do they affect human brain functions? "
Stastny J, Konstantinidis A, Schwarz MJ, Rosenthal NE, Vitouch O, Kasper S, Neumeister A. Biol Psychiatry. 2003 Feb 15;53(4):332-7. "Effects of tryptophan depletion and catecholamine depletion on immune parameters in patients with seasonal affective disorder in remission with light therapy."

## Claims

1. A method to diagnose, screen for or predict the development of an inflammatory disease in a subject, preferably a human subject, said method comprising determining the level of at least one, preferably at least two, more preferably at least three, most preferred at least four, inflammatory-specific gene product(s) in a biological sample isolated from said subject, preferably peripheral blood monocytes, wherein said gene is selected from the group comprising HSPC228, 34703_f_at, MCP-3, CCL2, EMP1, CDC42, TLE3, SPRY2, p40BBP, HSPC060, NAB2, HSPA1A, HSPA1B, MAPRE2 and OAS1.

2. A method according to claim 1, wherein said inflammatory disease is an auto-inflammatory disease, preferably a disease selected from the group comprising diabetes mellitus Type 1 (DM1), bipolar disorder (BP), rheumatoid arthritis (RA), multiple sclerosis (MS), psoriasis, Sjögren's syndrome, thyroid disease, systemic lupus erythematosus (SLE), scleroderma, and inflammatory bowel disease.

3. A method to diagnose, screen for or predict the development of an affective disorder (AD) in a subject, preferably a human subject, said method comprising determining the level of at least one, preferably at least two, more preferably at least three, most preferred at least four, AD-specific gene product(s) in a biological sample isolated from said subject, preferably peripheral blood monocytes, wherein said gene is selected from the group comprising CCR2, CX3CR1, DOK1, HBB, G-gamma globin, THBD, PHLDA1, DTR and GNLY.

4. A method according to any one of claims 1 to 3, wherein determining the level comprises determining the level of said at least one inflammatory-specific or AD-specific gene product in said isolated sample relative to the level of said at least one specific gene product in a control sample, preferably a biological sample isolated from a healthy subject.

5. A method according to any one of claims 1 to 4, wherein the level of said gene product is determined at the DNA or RNA level, preferably the mRNA level.

6. A method according to claim 5, wherein determining the level is performed by contacting a nucleic acid extract of said sample with at least one probe comprising a sequence that hybridises to a nucleotide region encoding an inflammatory-specific or AD-specific gene and, optionally, with at least one probe comprising a sequence that hybridises to a nucleotide encoding a housekeeping gene, wherein said at least one nucleotide probe is preferably immobilized on a solid support.

7. A method according to claim 6, wherein said nucleotide probes comprise DNA, RNA or cDNA and/or wherein said nucleic acid extract comprises nucleic acids with a detectable label.

8. A method according to any one of claims 1 to 4, wherein the level of said gene product is determined at the protein level.

9. A method according to claim 8, wherein determining the level is performed by contacting a protein extract of said sample with a specific binding partner of at least one protein encoded by an inflammatory- or AD-specific gene under conditions that allow formation of a complex between said binding partner and said protein and detecting complex formation.

10. A method according to claim 1 or 2, wherein the mRNA level of HSPC228, 34703_f_at, MCP-3, CCL2, EMP1, CDC42, TLE3, SPRY2, p40BBP, HSPC060, and/or NAB2, in a sample of said subject is at least two-fold higher and/or wherein the mRNA level of HSPA1A, HSPA1B, MAPRE2 and/or OAS1 is at least two-fold lower compared to the presence of said mRNA in a sample of a healthy subject.

11. A method according to claim 3, wherein the mRNA level of CCR2, CX3CR1 and/or DOK1 in a sample of said subject is at least two-fold lower and/or wherein the mRNA level of HBB, G-gamma globin, THBD, PHLDA1, DTR and/or GNLY is at least two-fold higher compared to the presence of said mRNA in a sample of a healthy subject.

12. A kit for diagnosing or screening for an inflammatory disease in a subject, comprising at least one reagent specifically reactive with an inflammatory-specific gene product selected from the group comprising HSPC228, 34703_f_at, MCP-3, CCL2, EMP1, CDC42, TLE3, SPRY2, p40BBP, HSPC060, NAB2, HSPA1A, HSPA1B, MAPRE2 and OAS1, optionally further comprising at least one reagent specifically reactive with a housekeeping gene product and/or a defined amount of an inflammatory-specific gene product.

13. A kit for diagnosing or screening for affective disorder (AD) in a subject, comprising at least one reagent specifically reactive with an AD-specific gene product selected from the group comprising CCR2, CX3CR1, DOK1, HBB, G-gamma globin, THBD, PHLDA1, DTR and GNLY, optionally further comprising at least one reagent specifically reactive with a housekeeping gene product and/or a defined amount of an AD-specific gene product.

14. A kit according to claim 12 or 13, wherein said reagent comprises an antibody or fragment thereof, or a nucleotide probe.

15. A kit according to any one of claims 12 to 14, wherein said at least one reagent is immobilized on a solid support, preferably a glass, nylon or nitrocellulose solid support.

16. An array of nucleotide probes comprising at least 10 nucleotide bases in length, wherein at least one probe hybridises to a fragment of at least one inflammatory-specific gene selected from the group comprising HSPC228, 34703_f_at, MCP-3, CCL2, EMP1, CDC42, TLE3, SPRY2, p40BBP, HSPC060, NAB2, HSPA1A, HSPA1B, MAPRE2 and OAS1, and optionally, wherein at least one probe hybridises to a fragment of at least one housekeeping gene.

17. Use of a method according to any one of claims 1, 2 and 9, a kit according to any claim 13 or an array according to claim 16 to diagnose or screen for increased risk of developing an inflammatory condition or disease, such as an auto-inflammatory disease, in a subject, preferably a human subject.

18. Use according to claim 17, wherein said inflammatory disease is selected from the group comprising Diabetes Mellitus type 1 (DM1), bipolar disorder (BP), rheumatoid arthritis (RA), multiple sclerosis (MS), Sjögren's syndrome, systemic lupus erythematosus (SLE), and inflammatory bowel disease.

19. An array of nucleotide probes comprising at least 10 nucleotide bases in length, wherein at least one probe hybridises to a fragment of at least one AD-specific gene selected from the group comprising CCR2, CX3CR1, DOK1, HBB, G-gamma globin, THBD, PHLDA1, DTR and GNLY, and optionally, wherein at least one probe hybridises to a fragment of at least one housekeeping gene.

20. Use of a method according to claim 3 or 9, a kit according to claim 12 or an array according to claim 19 to diagnose or screen for increased risk of developing an affective disorder in a subject, preferably a human subject.
